# EUROPEAN PATENT APPLICATION

(11) **EP 4 275 603 A1**
(43) Date of publication of application: **15.11.2023**
(21) Application number: 22736886.7
(22) Date of filing: 07.01.2022
(51) Int. Cl.: A61B 5/151, A61B 5/15

(54) **DISPOSABLE BLOOD COLLECTING DEVICE**

(30) Priority: 07.01.2021 KR 20210002008; 22.03.2021 KR 20210036866
(71) Applicant: Roahmed Co., Ltd., Changwon-Si, Gyeongsangnam-do 51573 (KR)
(72) Inventor: CHOI, Imcheol, Busan 46760 (KR)
(74) Representative: Gulde & Partner
(86) International application number: PCT/KR2022/000306
(87) International publication number: WO 2022/149909

(57) **Abstract**

A disposable blood lancet device according to the disclosure includes a barrel including a tubular barrel body; a tubular front slider inserted in the barrel, movable in an axial direction, and including a partial region of a front end exposed from the end of the barrel; a carrier body accommodated in the front slider and supporting the needle to protrude in a frontward direction; a needle carrier including a plurality of elastic transformation portion extending rearwards from the carrier body and elastically transformable inwards in a radial direction, and an interlocking holding projection protruding from the elastic transformation portion in a transverse direction to an axial direction, blocked by a rear end of the front slider, moving along with the front slider upon rearward movement of the front slider; an actuation spring elastically pushing the needle carrier in the frontward direction; and a trigger portion coming into contact with the elastic transformation portion upon the rearward movement of the needle carrier and elastically transforming the elastic transformation portion inwards in the radial direction so that the interlocking holding projection can be separated inwards from the rear end of the front slider to propel the needle carrier frontwards. Thus, tactile nerves are disturbed by the strike of the bumper spring coupled to the needle carrier as the needle carrier is blocked and released based on rectilinear motion, nociceptive nerves are blocked as the bumper spring is compressed by the inertial motion of the fired needle carrier and pressed against epidermis, and the needle is quickly pulled out by the resilience of the compressed bumper spring, thereby minimizing the pain and afterpain of a user.

## Description

### [TECHNICAL FIELD]

The disclosure relates to a disposable blood lancet device used in collecting blood from a skin surface.

### [BACKGROUND ART]

To perform a test using a small amount of blood, such as blood grouping and blood glucose measurement, a blood lancet device is used to pierce a skin surface with a needle. The blood lancet device is thrown away to prevent infection between users after it is used once.

In the skin puncturing device for patients (Korean patent No. 10-1177189) being widely used, a lancet body having a needle is accommodated in a tubular housing and elastically fired by an actuation spring so that the needle punctures the skin.

Meanwhile, another skin puncturing device for patients (Korean patent No. 10-1201034) discloses a structure in which, when a front end of a body is placed on a blood collection site and a push button is pressed in that direction, a moving member rises and a needle fixed therein is exposed to puncture the skin.

However, Korean patent No. 10-1177189 requires a series of two consecutive operations because the needle can be fired only by positioning the front end of the housing on the blood collection site and then pressing the push button provided at the rear end. Further, Korean Patent No. 10-1201 034 causes pain to last for a relatively long time because the needle remains pierced into the skin until a user intentionally removes the skin puncturing device.

### [DISCLOSURE]

### [TECHNICAL PROBLEM]

An aspect of the disclosure is to provide a disposable blood lancet device that can collect blood by only one simple operation.

Another aspect of the disclosure is to provide a disposable blood lancet device that can be inexpensively manufactured with minimal parts and a simple structure.

Still another aspect of the disclosure is to provide a disposable blood lancet device that can minimize pain caused by a needle while allowing the needle to be rapidly withdrawn.

Still another aspect of the disclosure is to provide a disposable blood lancet device that can keep a needle hygienic until the needle is used.

Still another aspect of the disclosure is to provide a disposable blood lancet device that can be effectively prevented from reuse.

Still another aspect of the disclosure is to provide a disposable blood lancet device that can prevent accidents caused by a used needle.

### [TECHNICAL SOLUTION]

According to an embodiment of the disclosure, a disposable blood lancet device includes: a tubular barrel including a front opening; a tubular front slider slidable within the barrel along an axial direction; a needle carrier including a carrier body including a needle protruding toward the front opening of the barrel and slidable within the front slider along the axial direction, at least one elastic transformation portion extending rearwards from the carrier body and elastically transformable inwards in a radial direction upon rearward movement, and an interlocking holding projection provided in the elastic transformation portion, blocking frontward movement of the carrier body relative to the front slider, and allowing the frontward movement of the carrier body upon the radially inward transformation of the elastic transformation portion; and an actuation spring accommodated in the barrel and elastically pushing the needle carrier in a frontward direction.

Further, when the interlocking holding projection is kept blocked by a stopper provided in the front slider, the needle carrier is caught in the rear end of the front slider and kept not to move frontwards.

Further, the stopper protrudes inwards in a radial direction of the front slider, and
a separation preventing protrusion is provided in the needle carrier and prevents the needle carrier from rearward separation as blocked by the stopper, so that the needle carrier introduced into the interior of said the front slider can be prevented from retracting by the separation preventing protrusion, thereby preventing the reuse of the needle carrier.

In addition, when a trigger portion is provided inside the barrel and comes into contact with the elastic transformation portion upon the rearward movement of the elastic transformation portion to elastically transform the elastic transformation portion inwards in the radial direction, the needle carrier may be inserted in the interior of the front slider by the movement of the needle carrier or the barrel.

Further, when a holding projection is provided on one of an inner wall of the barrel and an outer wall of the front slider, and a standby holding portion is provided on the other of the inner wall of the barrel and the outer wall of the front slider and engaging with the holding projection to stop and keep the front slider at a standby position, the front slider is prevented from separating in the frontward direction of the barrel.

Further, when a separation preventing portion provided at a position spaced frontwards or rearwards apart from the standby holding portion and blocked by the holding projection to stop and keep the front slider at a separation preventing position, the front slider is prevented from retracting to a certain extent or more in the rearward direction of the barrel.

In addition, when a bumper spring is mounted in a front of the needle carrier and extends longer than a protruding length of the needle, the buffer spring can reduce pain by striking epidermis before the needle and prevent accidents caused by the used needle by preventing the exposure of the needle after blood collection.

In addition, when the needle carrier includes a protective extending portion formed integrally with the carrier body to surround the needle and shaped like a rod extending along a longitudinal direction of the carrier body, the needle is prevented from damage and contamination until the protective extending portion is removed or until the needle is used.

According to an embodiment of the disclosure, a disposable blood lancet device includes: a tubular barrel including a front opening; a tubular front slider accommodated in the barrel; a needle carrier accommodated in the front slider and including a needle protruding toward the front opening of the barrel; and a holding projection provided on one of an inner wall of the barrel and an outer wall of the front slider, and a standby holding portion provided on the other of the inner wall of the barrel and the outer wall of the front slider and engaging with the holding projection to stop and keep the front slider at a standby position.

Further, when a separation preventing portion is provided at a position spaced frontwards or rearwards apart from the standby holding portion and blocked by the holding projection to stop and keep the front slider at a separation preventing position, the front slider is prevented from retracting to a certain extent or more in the rearward direction of the barrel.

In addition, when the needle carrier includes a plurality of elastic transformation portion extending rearwards from the carrier body and elastically transformable inwards in a radial direction; and an interlocking holding projection protruding from the elastic transformation portion in a transverse direction to an axial direction, blocked by a rear end of the front slider, moving along with the front slider upon rearward movement of the front slider, and separating inwards from the rear end of the front slider upon the radially inward transformation of the elastic transformation portion, the needle carrier is in a state of being caught in the rear end of the front slider by the interlocking holding protrusion and moved along with the front slider but can be inserted in the interior of the front slider when the elastic transformation portion is transformed inwards.

Further, when there are a stopper formed at the rear end of the front slider, and a separation preventing protrusion provided in the needle carrier and prevents the needle carrier from rearward separation as blocked by the stopper, the needle carrier is restricted from movement by the stopper and the interlocking holding protrusion provided in the front slider.

In addition, when there is a trigger portion provided inside the barrel and coming into contact with the elastic transformation portion upon the rearward movement of the elastic transformation portion to elastically transform the elastic transformation portion inwards in the radial direction, the needle carrier is inserted in the interior of the front slider by the movement of the needle carrier or the barrel.

In addition, when there is an actuation spring accommodated in the barrel and elastically pushing the needle carrier in a frontward direction, the needle carrier is propelled frontward by the elastic force of the spring, thereby enabling rapid and accurate blood collection.

In addition, when there is a bumper spring mounted in a front of the needle carrier and extending longer than a protruding length of the needle, the bumper spring can reduce pain by striking the epidermis before the needle and prevent accidents caused by the used needle by preventing the exposure of the needle after blood collection.

In addition, the needle carrier may include a protective extending portion formed integrally with the carrier body to surround the needle and shaped like a rod extending along a longitudinal direction of the carrier body, thereby preventing the needle from damage and contamination until the protective extending portion is removed or until the needle is used

In addition, the barrel may include a rear opening, and the disposable blood lancet device may further include a rear end cap blocking the rear opening, and at least one extension leg portion extending from the rear end cap along an interior of the barrel, thereby facilitating the assembly of the blood lancet device as the front slider and the needle carrier can be assembled even through the rear opening of the barrel.

In addition, when the trigger portion includes a slope provided on one of the rear end of the elastic transformation portion and the extension leg portion to transform the elastic transformation portion inwards upon the reward movement of the needle carrier, the trigger portion can be selectively provided in the barrel or the rear cap, thereby improving the structure of the barrel to be more simplified.

According to an embodiment of the disclosure, A disposable blood lancet device includes: a tubular barrel including a front opening; a tubular front slider accommodated in the barrel; a needle carrier accommodated in the front slider and including a needle protruding toward the front opening of the barrel; a bumper spring mounted in a front of the needle carrier and extending longer than a protruding length of the needle; and an elastic transformation portion extending rearwards from the needle carrier and elastically transformable with respect to the front slider.

### [ADVANTAGEOUS EFFECTS]

The blood lancet device according to the disclosure is capable of performing blood collection in a single, simple motion.

Further, according to embodiments of the disclosure, effects such as minimizing pain, maintaining hygiene, and preventing reuse are provided.

### [DESCRIPTION OF DRAWINGS]

FIG. 1 is a perspective view of a disposable blood lancet device according to the disclosure.
FIG. 2 is a longitudinal section view of a disposable blood lancet device according to the disclosure.
FIG. 3 is an exploded perspective view of a disposable blood lancet device according to the disclosure.
FIGS. 4 (a) to (f) are longitudinal section views showing sequential operations of using a disposable blood lancet device according to the disclosure.
FIG. 5 is a perspective view of a disposable blood lancet device according to another embodiment of the disclosure.
FIG. 6 is a longitudinal section view of a disposable blood lancet device according to another embodiment of the disclosure.
FIG. 7 is an exploded perspective view of a disposable blood lancet device according to another embodiment of the disclosure.
FIGS. 8 (a) to (f) of FIG. 8 are longitudinal section views showing sequential operations of using a disposable blood lancet device according to another embodiment of the disclosure.

### **DESCRIPTION OF REFERENCE NUMERALS**

| | | | |
|---|---|---|---|
| 1: | epidermis | 2: | needle hole |
| 10, 11: | blood lancet device | 100, 101: | barrel |
| 110, 111: | front opening | 120, 121: | standby holding portion |
| 130: | separation preventing portion | 140: | trigger portion |
| 141: | slope | 150: | rear opening |
| 160: | rear end cap holding grove | 200, 201: | front slider |
| 210, 211: | holding projection | 220, 221: | stopper |
| 230, 240: | guide cutout portion | 300: | needle carrier |
| 310: | carrier body | 320: | needle |
| 330: | elastic transformation portion | 340: | outward projection |
| 341: | interlocking holding protrusion | 342: | separation preventing protrusion |
| 350: | protective extending portion | 360: | actuation spring accommodating portion |
| 400: | rear end cap | 410: | extension leg portion |
| 411: | holding projection | 420: | actuation spring holder |
| 450: | trigger portion | 451: | slope |
| 500: | actuation spring | 700: | bumper spring |

### [BEST MODE]

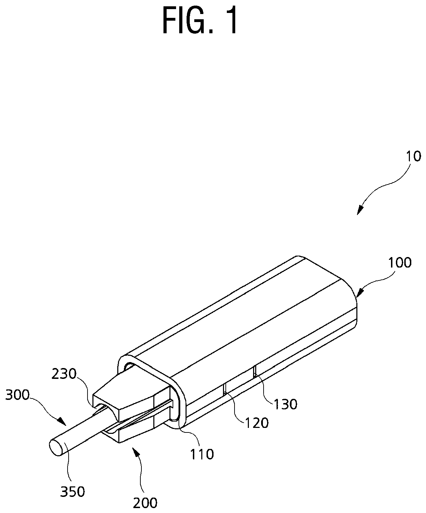

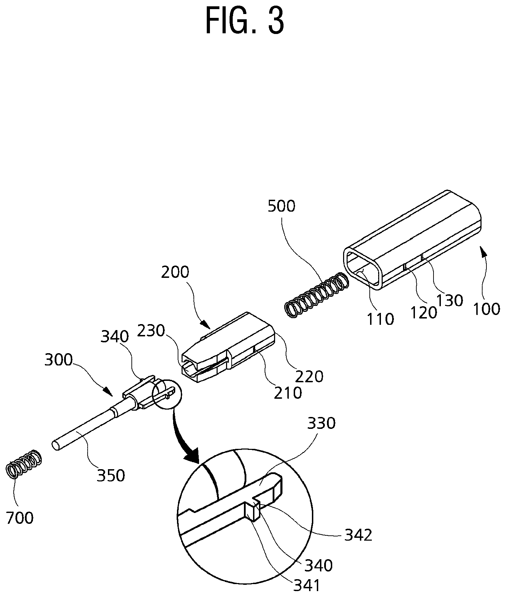

FIGS. 1 to 3 are a perspective view, a longitudinal section view, and an exploded perspective view of a disposable blood lancet device according to an embodiment of the disclosure. Details of a disposable blood lancet device according to an embodiment of the disclosure will be described with reference to FIGS. 1 to 3.

The disposable blood lancet device 10 according to the disclosure refers to a device that collects a small amount of blood by quickly forming a small needle hole 2 in epidermis 1. The blood lancet device 10 includes a tubular barrel 100, a front slider 200 inserted in the barrel 100, and a needle carrier 300 inserted in the front slider 200.

The barrel 100 refers to a tubular member that includes an open front end with a front opening 110, and a closed rear end. Inside the barrel 100, a standby holding portion 120 and a separation preventing portion 130 are located to limit the movement of the front slider 200. At a rear side of the barrel, a trigger portion 140 is formed.

The standby holding portion 120 prevents the front slider 200 from separating in a direction of the front opening 110 when the front slider 200 is inserted into the barrel 100. Due to the standby holding portion 120, only a free end of the front slider 200 inserted into the barrel 100 is exposed and the remaining portion is located inside the barrel 100 without exposure.

The separation preventing portion 130 limits a movement range of the front slider 200 inserted in the barrel 100 so that the front slider 200 can be prevented from being pushed further inwards from the position of the separation preventing portion 130 or separated in the direction of the front end as moving from the position of the standby holding portion 120 to the rear end of the barrel 100.

The trigger portion 140 refers to a portion that protrudes from an inner wall of the barrel 100 and limits a rearward movement of the needle carrier 300.

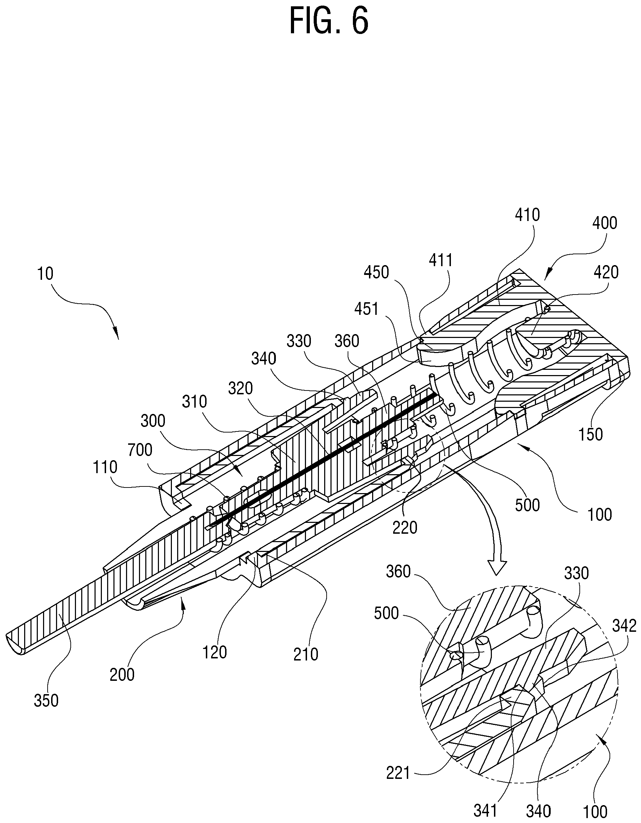

The front slider 200 refers to a tubular member that is accommodated in the barrel 100 and has a front end partially exposed to the outside through the front opening 110. The front slider 200 may be shaped to have a gradually increasing outer diameter from the front end toward the rear end. According to this embodiment, the front slider 200 is inserted in the barrel 100 through the front opening 110, and is, in a mounted state, prevented by the standby holding portion 120 provided inside the barrel 100 from being separating in the direction of the front end of the barrel 100, and allowed to slide only in the direction of the rear end of the barrel 100. The front slider 200 includes a holding projection 210 formed extending outwardly, a stopper 220 formed at the rear end of the front slider 200 or a stopper 221 (see FIG. 6) extending inwardly in an opening region at the rear end thereof, and a guide cutout portion 230 formed by partially cutting a front end portion of the front slider 200.

The holding projection 210 refers to a portion that protrudes from an outer surface of the front slider 200 and is blocked by the standby holding portion 120 and the separation preventing portion 130 of the barrel 100 to prevent the front slider 200 inserted in the barrel 100 from being separated.

Here, the holding projection is provided on the outer surface of the front slider, and the standby holding portion and the separation preventing portion are provided on the inner surface of the barrel. Alternatively, the holding projection may be provided on the inner surface of the barrel and the standby holding portion and the separation preventing portion may be provided on the outer surface of the front slider. Further, the separation preventing portion may be provided at a position spaced apart frontwards from the standby holding portion as well as the position spaced apart backwards from the standby holding portion. In addition, the holding projection may be implemented as a groove-shaped holding portion, or the standby holding portion and the separation preventing portion may be implemented as a standby holding projection and a separation preventing projection.

The stopper 220; 221 is formed at an end of a rear opening region of the front slider 200 or protrudes from the end in a central-axis direction of the front slider 200. The stopper 220; 221 is interlocked with a frontward movement of the needle carrier 300 and prevents a rear end portion of the needle carrier 300 from entering an inner region of the front slider 200 when the needle carrier 300 is positioned in an outer region through the rear opening region of the front slider 200. In addition, the stopper 220; 221 is interlocked with the rearward movement of the needle carrier 300 and serves to stop and prevent the needle carrier 300 from separating out from the front slider 200 when the needle carrier 300 is positioned in an inner region of the front slider 200.

The guide cutout portion 230 is formed in a front end region of the front slider 200 to have cut surfaces facing each other along a longitudinal direction, thereby preparing an opening in the front end region. The guide cutout portion 230 allows the front end portion of the front slider 200 to be elastically transformed. Therefore, the needle carrier 300 may be inserted and mounted even through the opening region at the front end of the front slider 200, and incorrect operations due to interference between the barrel 100 and the needle carrier 300 are prevented during the movement of the front slider 200 to thereby facilitate a sliding movement of the front slider 200. The presence of the guide cutout portion 230 is also advantageous to solve molding technical problems related to the overall shape of the front slider 200 and the formation of the stopper 220; 221.

The needle carrier 300 is movably accommodated at the rear end of the front slider 200 along an axial direction of the front slider 200. The needle carrier 300 includes a carrier body 310 formed to accommodate a needle 320 therein, and at least one elastic transformation portion 330 provided in an extension portion extended rearwards in the axial direction at the rear end of the carrier body 310. Here, there may be a pair or plurality of elastic transformation portions 330 symmetrical to an axial line.

The elastic transformation portion 330 is elastically transformable in a radial direction of the needle carrier 300 due to its shape. The elastic transformation portion 330 is provided with an outward projection 340 at the rear end thereof, and the outward projection 340 includes a front side protrusion functioning as an interlocking holding protrusion 341 that is blocked by the rear end of the front slider 200 or the stopper 220; 221. When the front slider 200 is moved rearwards by the interlocking between the interlocking holding protrusion 341 and the stopper 220; 221, the needle carrier 300 is also moved rearwards. On the rearward side of the outward projection 340, which is opposite to the interlocking holding protrusion 341 formed in the outward projection 340, a separation preventing protrusion 342 is formed. When the needle carrier 300 is being inserted in the front slider 200, the separation preventing protrusion 342 is blocked by the stopper 220; 221 so that the needle carrier 300 cannot move back and separate from the rear end of the front slider 200, thereby preventing the needle carrier 300 from rearward separation. Here, the interlocking holding protrusion and the separation preventing protrusion may be respectively formed in the elastic transformation portions separated from each other.

At the front end of the needle carrier 300, a protective extending portion 350 is formed long. The protective extending portion 350 may be formed integrally with a carrier body 310 upon the molding of the carrier body 310 while surrounding the needle 320. The protective extending portion 350 serves to hygienically protect the needle 320 until using the needle 320 and to prevent deformation of the needle 320. A manipulation tube (not shown) may be disposed to externally cover a connection portion between the protective extending portion 350 and the carrier body 310. The manipulation tube (not shown) may resist external forces acting from the protective extending portion 350 in the radial direction, thereby protecting the protective extending portion 350 not to be easily removed until the manipulation tube (not shown) is removed.

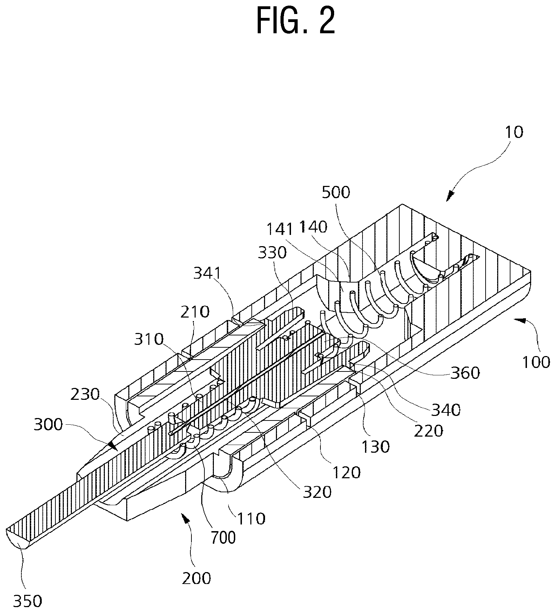

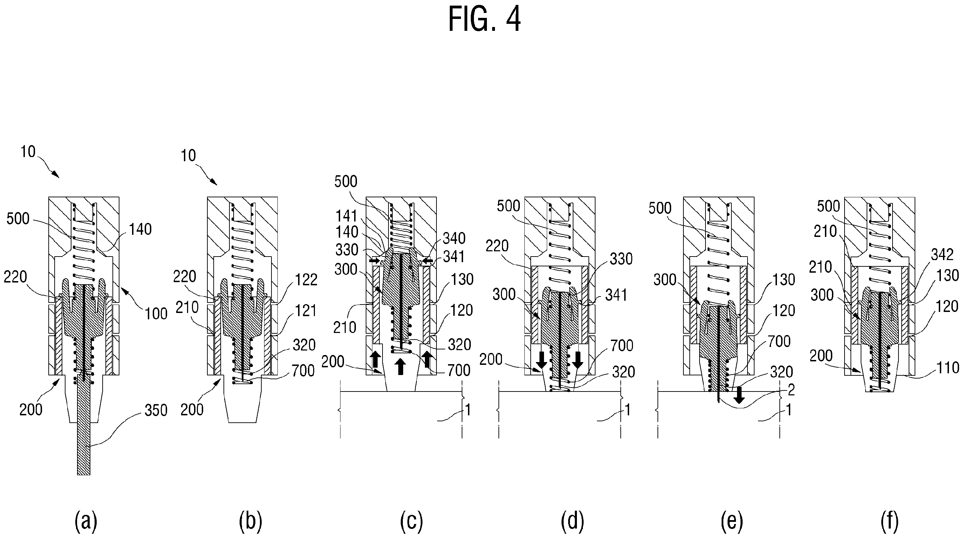

The operations of using the blood lancet device 10 in the standby state as shown in FIGS. 1 and 2 will be described with reference to (a) to (f) of FIG. 4.
(a) of FIG. 4 is a sectional view showing the standby state of the assembled blood lancet device 10 according to the disclosure. The protective extending portion 350 is exposed from the front end portion of the barrel 100, and the front end portion of the holding projection 210 is blocked by the standby holding portion 120 from moving forwards, thereby preventing the frontward movement of the front slider 200. In this standby holding state, the protective extending portion 350 can be easily removed by pulling it forward.
(b) of FIG. 4(b) illustrates the blood lancet device 10 from which the protective extending portion 350 is removed. The removed protective extending portion 350 is discarded, and the removal of the protective extending portion 350 causes the needle 320 for blood collection to be exposed at the front of the needle carrier 300 inside the front slider 200. In this state, when the barrel 100 is pressurized after putting the front end of the blood lancet device 10, i.e., the front end of the front slider 200, on the epidermis 1 of a person to be subjected to blood sampling, the front slider 200 is moved toward the rear end of the barrel 100.
(c) of FIG. 4 illustrates a state that the front slider 200 is moved rearwards inside the barrel 100. Here, the needle carrier 300 is about to be fired in the forward direction. When the front slider 200 is moved in the rearward direction, the holding projection 210 is elastically transformed due to its shape and moved to the rearward direction without difficulty. As the front slider 200 is moved rearwards, the holding projection 210 separates from the standby holding portion 120 and moves to the separation preventing portion 130, thereby keeping the front slider 200 stopped.

Meanwhile, the engagement between the rear end of the front slider 200 and the outward projection 340 causes the needle carrier 300 to also move in the rearward direction. When the needle carrier 300 moves in the rearward direction of the barrel 100, the elastic transformation portion 330 formed integrally with the needle carrier 300 comes into contact with the trigger portion 140 provided on the inner wall of the barrel 100, and is elastically transformed as being guided to the inward side of the barrel 100 by a slope 141 provided on the trigger portion 140. When the elastic transformation portion 330 is elastically transformed to a certain extent or more, a width between the opposite outward projections 340 becomes narrower than the inner width of the rear opening region of the front slider 200, so that the needle carrier 300 can be introduced into the front slider 200 and move in the front end direction without a blocking engagement between the interlocking holding protrusion 341 formed in the outward projection 340 and the stopper 220; 221.

Meanwhile, as the needle carrier 300 moves along with the front slider 200 in the rearward direction, the actuation spring 500 supported at the rear end of the barrel 100 is compressed, so that the elastic force of the compressed actuation spring 500 can generate a strong driving force, thereby making the needle carrier 300 move down and reach the epidermis 1.

(d) of FIG. 4 illustrates the moment when the needle carrier 300 is fired frontwards by the compressed actuation spring 500 and touches the epidermis 1. The needle carrier 300 is moved rectilinearly up to the epidermis 1 by the elastic force of the actuation spring 500, and a bumper spring 700 provided in the front of the carrier body 310 strikes the epidermis 1. After the bumper spring 700 strikes the epidermis 1, the bumper spring 700 is compressed by the continued inertial motion of the needle carrier 300 and stores resilience for retraction of the needle carrier 300.

(e) of FIG. 4 illustrates that the blood collection needle 320 penetrates into the epidermis 1 and forms a needle hole 2. In this case, the blood collection needle 320 for the penetration is rectilinearly moved with the needle carrier 300 by the elastic force of the actuation spring 500. When the needle carrier 300 is moved rectilinearly, the needle carrier 300 is fired with the minimum transverse movement from the central axis and thus forms the needle hole 2 at an exactly desired point without shaking.

When the bumper spring 700 is compressed to a certain extent or more by the inertial force applied from the needle carrier 300, the needle 320 for the blood collection is propelled backward by the resilience of the bumper spring 700 and thus returns from the epidermis 1 to the inside of the barrel 100, that is, the inside of the front slider 200.

After these operations, the blood collection needle 320, which has received the resilience of the compressed bumper spring 700, is quickly pulled out of the epidermis 1, returns to the position (d) of FIG. 4, and is prevented from being exposed to the outside of the barrel 100. In this case, the outward projection 340 of the needle carrier 300 is not separated in the rearward direction of the barrel 100 as being blocked by the stopper 220;221 formed at the rear end of the front slider 200, and the front slider 200 is prevented from moving in the frontward direction of the barrel 100 as being pushed into a position where the holding projection 210 formed on the outer side of the front slider 200 blocked by the separation preventing portion 130 of the barrel 100, thereby preventing the needle carrier 300 kept inside the front slider 200 from being separated from the front slider 200 and being exposed to the outside. Further, the needle carrier 300 may be kept supported on the inner wall of the front slider 200 by the elastic force of the elastic transformation portion 330.

(f) of FIG. 4 illustrates a state that the needle carrier 300 cannot be reused after the disposable blood lancet device 10 has been used once. Because the front slider 200 of the disposable blood lancet device 10, which has been used once, is kept caught at a stop position by the stopper 220; 221 and the separation preventing portion 130, the front slider 200, which has been once inserted into the separation preventing portion 130, cannot be moved toward the front opening 110.

Further, because the outward projection 340 of the needle carrier 300 cannot get out as being blocked by the rear protrusion, i.e., the stopper 220; 221 of the front slider 200, the needle carrier 300 cannot be reloaded as being caught again by the rear protrusion, and thus the blood lancet device 10 becomes non-reusable.

With this configuration, the blood lancet device 10 includes the minimum number of parts necessary for blood collection. When the barrel 100 is pressed after removing the protective extending portion 350 and positioning the blood lancet device 10 at a blood collection point, the front slider 200 retracts into the barrel 100 and moves back along with the needle carrier 300. As a result of this movement, the needle carrier 300 is separated from the front slider 200 by the trigger portion 140 and moves forward to the front opening 110 of the barrel 100, thereby performing the blood collection. This operation of the blood lancet device may be supplemented by the actuation spring 500 and the bumper spring 700 (to be described later). The actuation spring 500 is inserted into the inward rear end of the barrel 100. The actuation spring 500 has a first side supported on the barrel 100 and a second side coupling with an actuation spring accommodating portion 360 present at the rear end of the needle carrier 300. The actuation spring 500 connected as above always elastically presses the needle carrier 300. By using the elastic force of the actuation spring 500, the needle carrier 300 can be quickly propelled toward the front end portion of the barrel 100. However, the needle carrier 300 at a position initially coupling with the front slider 200 is restricted from frontward movement because the interlocking holding protrusion 341 is blocked by the stopper 220; 221 of the front slider 200. Therefore, the needle carrier 300 resists the pressurizing force of the actuation spring 500 and remains in its mounting position. The actuation spring 500 may be made of an elastic material such as a leaf spring, and silicone, but may be provided in the form of a compressive coil spring.

Meanwhile, the front end of the needle carrier 300 is mounted with the bumper spring 700 surrounding the needle 320 and the protective extending portion 350. The bumper spring 700 extends further in the frontward direction than the needle 320, and thus strikes and presses against a user's skin before the needle 320 when the needle carrier 300 is driven for the blood collection in the frontward direction of the barrel 100. When the bumper spring 700 touches the epidermis of the user, it strikes and disrupts tactile nerves around the blood collection point. As the bumper spring 700 is compressed, it compresses the periphery of the epidermis to be punctured by the needle 320, thereby blocking nociceptive nerves around the epidermis. While the tactile nerves are disturbed and the nociceptive nerves are blocked as above, the epidermis is punctured by the needle 320 to make a user feel less pain. The compressed bumper spring 700 returns to its original state by generating the resilience. Thus, the needle 320 is quickly pulled out of the epidermis, thereby reducing pain during such a pulling-out operation. The bumper spring 700 may be provided in the form of a compressive coil spring as shown, but in some cases may be provided in the form of an elastic material such as a leaf spring, and silicone.

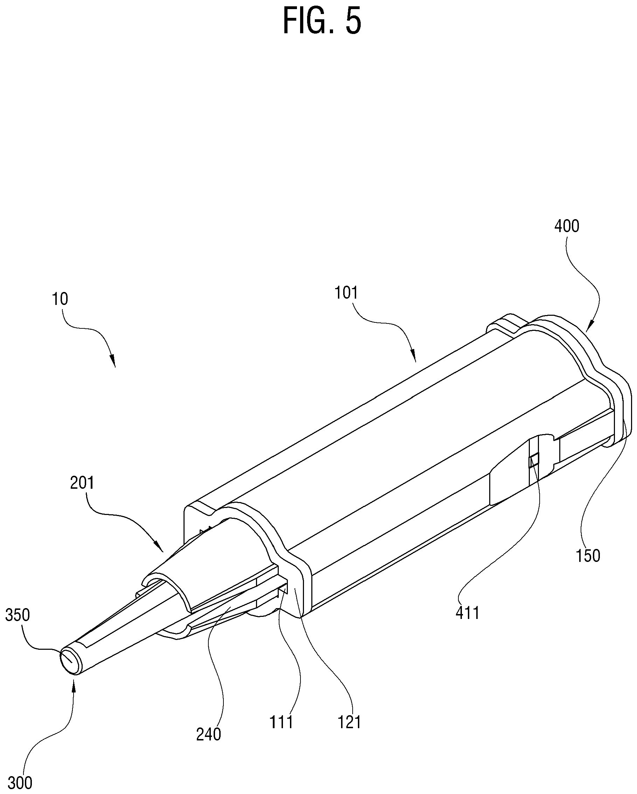

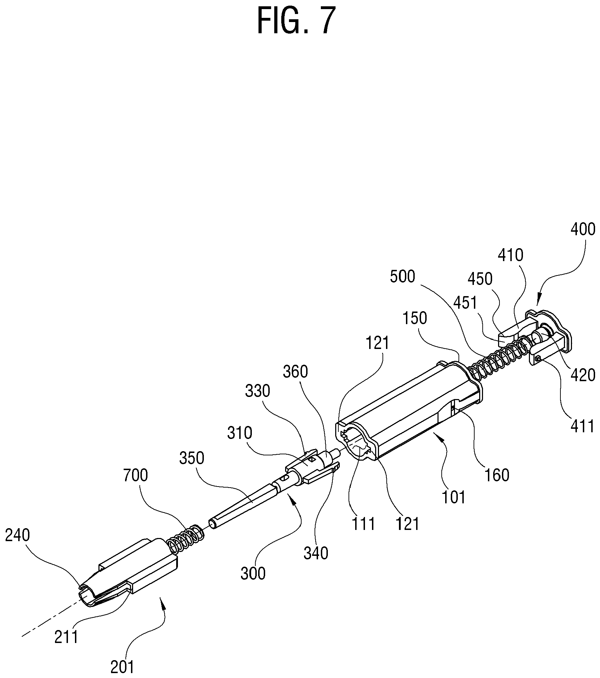

FIGS. 5 to FIG. 7 illustrate a configuration of a blood lancet device according to another embodiment of the disclosure. In this embodiment, a blood lancet device 11 includes a tubular barrel 101, a front slider 201 inserted into the barrel 101, and a needle carrier 300 inserted into the front slider 201 and provided with a needle 320.

The barrel 101 is a tubular member opened at both ends thereof with a front opening 111 at a front end portion and a rear opening 150 at a rear end portion. The barrel 101 is formed with a standby holding portion 121 that restricts the movement of the front slider 201. The standby holding portion 121 is formed in proximity to the front opening 111 to prevent the front slider 201 from separating in the front end portion. In addition, a rear end cap holding grove 160 is formed at an inward rear end of the barrel 101 to accommodate a rear end cap 400 (to be described later) therein.

The rear opening 150 allows parts to be inserted and assembled even through the rear end of the barrel 101, thereby facilitating the assembly of the parts. The rear opening 150 is closed by the rear end cap 400 (to be described later). In some cases, the barrel 101 may be formed symmetrically with respect to a midpoint between the front opening 111 and the rear opening 150, thereby solving a problem of misassembly due to indistinguishableness between the front opening 111 and the rear opening 150.

The front slider 201 is accommodated in the barrel 101. The front slider 201 has a front end partially exposed to the outside through the front opening 111. The front slider 201 has the shape of a tubular body along the axial direction of the barrel 101, and has an outer diameter that gradually increases from the front end to the rear end. Therefore, the front slider 201 is inserted even through the rear opening 150 during the assembly.

Like the embodiment shown in FIGS. 1 to 4, the front slider 201 may further have a holding projection 211 formed on its outer side. Accordingly, the holding projection 211 causes a portion of the front slider 201 to protrude outwardly and be blocked by the standby holding portion 121 formed in the barrel 101, thereby preventing the front slider 201 from separating in the direction of the front end of the barrel 101. In addition, like the embodiment shown in FIGS. 1 to 4, a separation preventing portion 130 may be further formed on the inner side of the barrel 101.

Further, a stopper 221 is formed in the rear opening region of the front slider 201. The stopper 221 refers to a portion protruding in the direction of the central axis of the front slider 201. The stopper 221 is interlocked with each of an interlocking holding protrusion 341 and a separation preventing protrusion 342 of the needle carrier 300 (to be described later). When the needle carrier 300 is located at the rear end of the front slider 201, the stopper 221 is interlocked with the frontward movement of the needle carrier 300 and prevents a rear end portion of the needle carrier 300 from entering the inner region of the front slider 201. When the needle carrier 300 entirely enters the inner region of the front slider 201, the stopper 221 is interlocked with the rearward movement of the needle carrier 300 to prevent the needle carrier 300 from moving out of the front slider 201.

The guide cutout portions 240 are formed in a front end region of the front slider 201 to face each other along a longitudinal direction. The guide cutout portion 240 facilitates the forward and backward movement of the needle carrier 300 when the needle carrier 300 is driven inside the front slider 201. Further, the guide cutout portion 240 is also advantageous to solve molding technical problems related to the tapered shape of the front slider 201 and the formation of the stopper 221.

Inside the front slider 201, the needle carrier 300 is movably accommodated along the axial direction. The needle carrier 300 has a carrier body 310 present inside the front slider 201.

The carrier body 310 is mounted with the blood collection needle 320 at the front end thereof, and includes a pair of elastic transformation portions 330 provided at the rear end thereof and extending rearwards in axial symmetry.

These elastic transformation portions 330 are elastically transformable in a radial direction. The elastic transformation proportion 330 is provided with an outward projection 340 at the rear end thereof, and the outward projection 340 includes a front side protrusion functioning as the interlocking holding protrusion 341 that is blocked by the rear end of the front slider 201. When the front slider 200 is moved rearwards by the interlocking of the interlocking holding protrusion 341, the needle carrier 300 also moves rearwards along with the front slider 201. On the opposite side of the interlocking holding protrusion 341 formed in the outward projection 340, the separation preventing protrusion 342 is formed. When the needle carrier 300 is being inserted in the front slider 201, the separation preventing protrusion 342 is blocked by the stopper 221 so that the needle carrier 300 cannot move back and separate from the rear end of the front slider 201, thereby preventing the needle carrier 300 from rearward separation.

At the front end of the needle carrier 300, a protective extending portion 350 is extended long in the direction of the front end. The protective extending portion is formed integrally with the carrier body 310 upon the molding of the carrier body 310 while surrounding the needle 320. The protective extending portion 350 serves to hygienically protect the blood collection needle 320 and to prevent the risk of undesired incorrect operations. The protective extending portion 350 may be protected from the outside by a manipulation tube (not shown) that covers the exterior of the protective extending portion 350.

The rear end cap 400 covers the rear opening 150 of the barrel 101 to prevent the front slider 201 and the needle carrier 300 inserted in the barrel 101 from separating through the rear opening 150. The rear end cap 400 has an extension leg portion 410 extending into the interior of the barrel 101. The extension leg portion 410 is provided with a holding projection 411 at a side portion thereof, and the holding projection 411 is caught in the rear end cap holding grove 160 formed on the wall of the barrel 101 to prevent the rear end cap 400 from separation. When the holding projection 411 and the rear end cap holding groove 160 are engaged, the rear end cap 400 and the barrel 101 are strongly coupled together, thereby preventing the front slider 201 and the needle carrier 300 present therein from the rearward separation.

At the center of the rear end cap 400, an actuation spring holder 420 is formed to accommodate the actuation spring 500 (to be described later). The actuation spring holder 420 couples with one end of the actuation spring 500 (to be described later), and the other end of the actuation spring 500 is coupled to an actuation spring accommodating portion 360 present at the rear end of the needle carrier 300.

Meanwhile, a terminal region of the extension leg portion 410 may be provided with a trigger portion 450, i.e., a slope that is gradually inclined inwards from the front end. The trigger portion 450 formed in this case has a slope 451 that is inclined along the surface. The slope 451 of the trigger portion 450 comes into contact with the rear end of the elastic transformation portion 330 when the needle carrier 300 moves in the rearward direction, thereby causing the elastic transformation portion 330 to be elastically transformed inwards in the radial direction. Thus, when the elastic transformation portion 330 is elastically transformed inwards, the interlocking holding protrusion 341 forms a narrower width than the stopper 221 and is then introduced inwards. Then, the interlocking state between the interlocking holding protrusion 341 and the stopper 221 is released, and the needle carrier 300 is fired in the front direction by the elastic force of the actuation spring 500. Meanwhile, the trigger portion 450 may be formed inside the barrel 101 like that according to the foregoing embodiment shown in FIGS. 1 to 4. When the trigger portion 450 is formed inside the barrel 101, its shape may be relatively complicated along with increase in the weight of the barrel, but the shape of the rear end cap 400 may be simplified. On the other hand, when the trigger portion 450 is formed in the rear end cap 400, the interior of the barrel 101 may be simplified to improve the structure of the barrel 101 and increase productivity.

The actuation spring 500 is interposed between the needle carrier 300 and the rear end cap 400, and the actuation spring 500 elastically presses the needle carrier 300 always. In this case, the interlocking holding protrusion 341 of the needle carrier 300 is blocked by the stopper 221 of the front slider 201 and restricted from its frontward movement, and thus the needle carrier 300 keeps its mounting position while resisting the pressing force of the actuation spring 500.

The front end of the needle carrier 300 is mounted with the bumper spring 700 surrounding the needle 320 and the protective extending portion 350. The bumper spring 700 extends further in the frontward direction than the needle 320, and thus strikes and presses against a user's skin before the needle 320 when the needle carrier 300 is driven for the blood collection in the frontward direction of the barrel 101. When the bumper spring 700 touches the epidermis of the user, it strikes and disrupts tactile nerves around the blood collection point. As the bumper spring 700 is compressed, it compresses the periphery of the epidermis to be punctured by the needle 320, thereby blocking nociceptive nerves around the epidermis. While the tactile nerves are disturbed and the nociceptive nerves are blocked as above, the epidermis is punctured by the needle 320 to make a user feel less pain. The compressed bumper spring 700 returns to its original state by generating the resilience. Thus, the needle 320 is quickly pulled out of the epidermis, thereby reducing pain during such a pulling-out operation. The bumper spring 700 may be provided in the form of a compressive coil spring as shown, but in some cases may be provided in the form of an elastic material such as a leaf spring, and silicone.

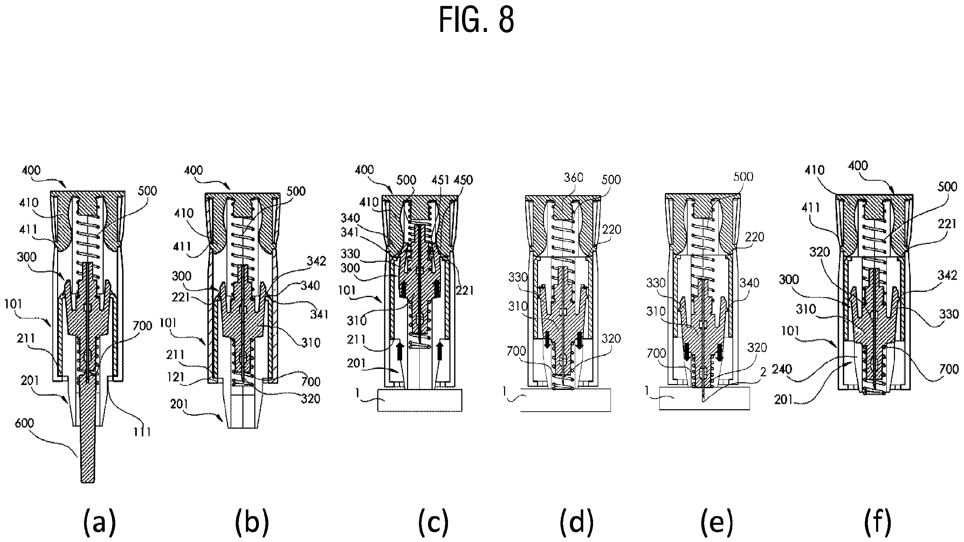

FIGS. 8 (a) to (f) are longitudinal section views showing sequential operations of using the disposable blood lancet device 11 according to the disclosure.

Referring to (a) of FIG. 8, the front slider 201 and the needle carrier 300 are inserted inside the barrel 101 of the disposable blood lancet device 11 according to the disclosure, and the protective extending portion 350 is exposed to the outside of the disposable blood lancet device 11 through the front opening region of the front slider 201. Thus, a user can easily separate the protective extending portion 350 from the needle carrier 300.

Referring to (b) of FIG. 8, when the protective extending portion 350 separated as above is removed from the needle carrier 300, the front end of the needle 320 is exposed to the interior of the front slider 201 and ready for blood collection. Before firing, the needle carrier 300 is interlocked to move along with the rearward movement of the front slider 201 by the engagement between the interlocking holding protrusion 341 and the stopper 221.

(c) of FIG. 8 shows a state just before the loaded needle carrier 300 is fired to a person to be subjected to blood sampling, in which, when the disposable blood lancet device 11 is positioned on the epidermis of that person and the barrel 101 is pressurized, the front slider 201 moves rearwards inside the barrel 101 and the needle carrier 300 also moves in the rearward direction by the engagement between the stopper 221 and the interlocking holding protrusion 341. In this case, the elastic transformation portion 330 formed integrally with the needle carrier 300 rises by the rising needle carrier 300 along with the rising of the front slider 201, and is elastically transformed as being guided inwards by the slope 451 of the trigger portion 450. When the elastic transformation portion 330 is elastically transformed to a certain extent or more, the width end of the interlocking holding protrusion 341 becomes narrower than the inner diameter of the stopper 221 so that the needle carrier 300 can be inserted in the front slider 201. As the needle carrier 300 rises, the actuation spring 500 is compressed, so that the elastic force of the compressed actuation spring 500 can generate a strong driving force, thereby making the needle carrier 300 move down and reach the epidermis 1.

(d) of FIG. 8 illustrates an operation that the needle carrier 300 is being fired forward by the compressed actuation spring 500. When the needle carrier 300 is fired downward at the moment when the elastic transformation portion 330 of the needle carrier 300 enters the interior of the front slider 201, the needle carrier 300 rectilinearly moves until touching the epidermis, and a bumper spring 700 provided in the front of the carrier body 310 strikes the epidermis 1 first. After the bumper spring 700 strikes the epidermis 1, the bumper spring 700 is compressed by the continued inertial motion of the needle carrier 300.

(e) of FIG. 8 shows that the needle carrier 300 compresses the bumper spring 700 and the needle 320 is inserted into the epidermis 1 to form a needle hole 2 and enable blood collection. The needle 320 is driven to be inserted by the rectilinear motion of the needle carrier 300, thereby forming the needle hole 2 exactly at a desired point without shaking, and minimizing the transverse movement from the central axis of the needle carrier 300. When the bumper spring 700 is compressed to a certain extent or more by the inertial force applied from the needle carrier 300, the needle 320 of the needle carrier 300 is pulled out of the epidermis 1 in the rearward direction by the resilience of the bumper spring 700 and thus returns to the interior of the front slider 201.

After these operations, the needle 320 of the needle carrier 300, which has received the resilience of the compressed bumper spring 700, is quickly pulled out of the epidermis 1 and returns to the position (d) of FIG. 8, and the needle carrier 300 is not exposed to the outside because the needle carrier 300 cannot get out of the interior of the front slider 201.

(f) of FIG. 8 illustrates detailed operations of the stopper 221 and the separation preventing protrusion 342 of the disposable blood lancet device 11 according to the disclosure, and shows that the needle carrier 300 cannot be reused after the disposable blood lancet device 11 has been used once.

The front slider 201 of the disposable blood lancet device 11, which has been used once, enters the interior of the barrel 101, and is not separable even though an attempt is made to forcibly separate the front slider 201 toward the front opening 110 of the barrel 101 because it is caught in in the standby holding portion 121 of the barrel 101. Further, the needle carrier 300 is not interlocked with the movement of the front slider 201 after the elastic transformation portion 330, the interlocking holding protrusion 341 and the separation preventing protrusion 342 of the needle carrier 300 are fired into the interior of the front slider 201, so that the front slider 201 cannot move the needle carrier 300 upwards to press the actuation spring 500. Even though the needle carrier 300 is forcibly moved, the needle carrier 300 cannot get out of the interior of the front slider 201 due to the engagement between the stopper 221 and the separation preventing protrusion 342. Therefore, the needle 320 is not reusable because it is not exposed to the outside of the front slider 201.

According to the foregoing disposable blood lancet device 11, the actuation spring 500 is compressed by the interlocking of the front slider 201 and the needle carrier 300, and the needle carrier 300 is fired in a straight direction to minimizes transverse movement, thereby minimizing the pain of a person to be subjected to blood sampling.

Further, the pain of a user can be additionally reduced because the bumper spring 700 striking the periphery of the blood collection point disturbs the tactile nerves at the striking point and blocks the nociceptive nerves around a site punctured by the needle 320, and the pain can be additionally reduced because the needle 320 is quickly pulled out of the epidermis 1 by the resilience of the compressed bumper spring 700.

In addition, the protective extending portion 350 allows the needle to remain hygienic until use.

When the needle carrier 300 is introduced into the front slider 201 after being used, the reuse of the disposable blood lancet device 11 is effectively prevented due to the operations of the separation preventing protrusion 342 and the stopper 221, and injury or infection caused by the used needle is prevented because the bumper spring 700 extending longer than the needle 320 prevents the used needle 320 from being exposed to the outside.

## Claims

1. A disposable blood lancet device comprising:
a tubular barrel comprising a front opening;
a tubular front slider slidable within the barrel along an axial direction;
a needle carrier comprising a carrier body comprising a needle protruding toward the front opening of the barrel and slidable within the front slider along the axial direction, at least one elastic transformation portion extending rearwards from the carrier body and elastically transformable inwards in a radial direction upon rearward movement, and an interlocking holding projection provided in the elastic transformation portion, blocking frontward movement of the carrier body relative to the front slider, and allowing the frontward movement of the carrier body upon the radially inward transformation of the elastic transformation portion; and
an actuation spring accommodated in the barrel and elastically pushing the needle carrier in a frontward direction.

2. The disposable blood lancet device of claim 1, wherein the interlocking holding projection is kept blocked by a stopper provided in the front slider.

3. The disposable blood lancet device of claim 2, wherein
the stopper protrudes inwards in a radial direction of the front slider, and
the disposable blood lancet device further comprises a separation preventing protrusion provided in the needle carrier and preventing the needle carrier from rearward separation as blocked by the stopper.

4. The disposable blood lancet device of claim 1, further comprising a trigger portion provided inside the barrel and coming into contact with the elastic transformation portion upon the rearward movement of the elastic transformation portion to elastically transform the elastic transformation portion inwards in the radial direction.

5. The disposable blood lancet device of claim 1, further comprising a holding projection provided on one of an inner wall of the barrel and an outer wall of the front slider, and a standby holding portion provided on the other of the inner wall of the barrel and the outer wall of the front slider and engaging with the holding projection to stop and keep the front slider at a standby position.

6. The disposable blood lancet device of claim 5, further comprising a separation preventing portion provided at a position spaced frontwards or rearwards apart from the standby holding portion and blocked by the holding projection to stop and keep the front slider at a separation preventing position.

7. The disposable blood lancet device of claim 1, further comprising a bumper spring mounted in a front of the needle carrier and extending longer than a protruding length of the needle.

8. The disposable blood lancet device of claim 1, wherein the needle carrier comprises a protective extending portion formed integrally with the carrier body to surround the needle and shaped like a rod extending along a longitudinal direction of the carrier body.

9. A disposable blood lancet device comprising:
a tubular barrel comprising a front opening;
a tubular front slider accommodated in the barrel;
a needle carrier accommodated in the front slider and comprising a needle protruding toward the front opening of the barrel; and
a holding projection provided on one of an inner wall of the barrel and an outer wall of the front slider, and a standby holding portion provided on the other of the inner wall of the barrel and the outer wall of the front slider and engaging with the holding projection to stop and keep the front slider at a standby position.

10. The disposable blood lancet device of claim 9, further comprising a separation preventing portion provided at a position spaced frontwards or rearwards apart from the standby holding portion and blocked by the holding projection to stop and keep the front slider at a separation preventing position.

11. The disposable blood lancet device of claim 9, wherein the needle carrier comprises a plurality of elastic transformation portion extending rearwards from a carrier body and elastically transformable inwards in a radial direction; and an interlocking holding projection protruding from the elastic transformation portion in a transverse direction to an axial direction, blocked by a rear end of the front slider, moving along with the front slider upon rearward movement of the front slider, and separating inwards from the rear end of the front slider upon the radially inward transformation of the elastic transformation portion.

12. The disposable blood lancet device of claim 11, further comprising:
a stopper formed at the rear end of the front slider, and
a separation preventing protrusion provided in the needle carrier and preventing the needle carrier from rearward separation as blocked by the stopper.

13. The disposable blood lancet device of claim 11, further comprising a trigger portion provided inside the barrel and coming into contact with the elastic transformation portion upon the rearward movement of the elastic transformation portion to elastically transform the elastic transformation portion inwards in the radial direction.

14. The disposable blood lancet device of claim 9, further comprising an actuation spring accommodated in the barrel and elastically pushing the needle carrier in a frontward direction.

15. The disposable blood lancet device of claim 9, further comprising a bumper spring mounted in a front of the needle carrier and extending longer than a protruding length of the needle.

16. The disposable blood lancet device of claim 9, wherein the needle carrier comprises a protective extending portion formed integrally with the carrier body to surround the needle and shaped like a rod extending along a longitudinal direction of the carrier body.

17. The disposable blood lancet device of claim 9 or 13, wherein
the barrel comprises a rear opening, and
the disposable blood lancet device further comprises a rear end cap blocking the rear opening, and at least one extension leg portion extending from the rear end cap along an interior of the barrel.

18. The disposable blood lancet device of claim 17, wherein the trigger portion comprises a slope provided on one of the rear end of the elastic transformation portion and the extension leg portion to transform the elastic transformation portion inwards upon the reward movement of the needle carrier.

19. A disposable blood lancet device comprising:
a tubular barrel comprising a front opening;
a tubular front slider accommodated in the barrel;
a needle carrier accommodated in the front slider and comprising a needle protruding toward the front opening of the barrel;
a bumper spring mounted in a front of the needle carrier and extending longer than a protruding length of the needle; and
an elastic transformation portion extending rearwards from the needle carrier and elastically transformable with respect to the front slider.
